# FASCICULE DE BREVET EUROPEEN

(11) **EP 3 443 947 B1**
(45) Date de publication et mention de la délivrance du brevet: **29.09.2021**
(21) Numéro de dépôt: 17186302.0
(22) Date de dépôt: 15.08.2017
(51) Int. Cl.: A61J 1/20, A61M 5/20, A61M 5/315, A61M 5/168, A61M 5/172

(54) **DISPOSITIF POUR PRÉPARER UNE DOSE DE QUANTITÉ DE MATIÈRE**
VORRICHTUNG ZUM VORBEREITEN EINER DOSIS EINER STOFFMENGE
DEVICE FOR PREPARING A DOSE OF MATERIAL

(43) Date de publication de la demande: 20.02.2019
(73) Titulaire: Prodictis SA, 1003 Lausanne (CH)
(72) Inventeur: Schwartz, Jean-Jacques, 1005 Lausanne (CH)
(74) Mandataire: P&TS SA (AG, Ltd.)

(56) Documents cités:
- EP-A2- 1 391 734
- GB-A- 1 335 797
- US-A1- 2013 310 743
- US-B1- 6 213 354
- US-B1- 6 604 908

## Description

### Domaine technique

La présente invention concerne un dispositif pour préparer une dose de matière ainsi qu'un procédé pour préparer ladite dose de matière.

### Etat de la technique

De nombreux procédés industriels comprennent une étape de dosage d'un produit pour obtenir une composition avec une concentration précise du produit, par exemple pour la fabrication d'un médicament dans l'industrie pharmaceutique. Pour cela, il est essentiel de disposer d'un dispositif fiable pour mesurer une quantité ad hoc de produit.

L'étape de dosage est également essentielle lorsqu'il s'agit de dispositifs médicaux qui administrent une formulation injectable qui sont injecté directement au patient. Ce type de dispositif doit permettre la délivrance d'une quantité constante et répétée de produit.

On connait des dispositifs de dosage utilisant un vérin, notamment un vérin pneumatique. Le document US4778451 décrit un dispositif de délivrance d'un produit pharmaceutique injectable par perfusion. Le produit est dans une poche comprise dans une enceinte étanche qui est mise sous pression par un vérin pneumatique, ici une seringue pour délivrer le produit au patient. La seringue comprend un piston qui se déplace dans un cylindre qui débouche sur l'enceinte, de sorte que le déplacement du piston dans le cylindre fait varier le volume de l'ensemble enceinte et cylindre, ce qui fait varier la pression qui s'exerce sur la poche.

Dans le dispositif selon ce document, le médecin actionne le piston pour augmenter la pression dans l'enceinte et comprimer la poche, ce qui permet d'éjecter une certaine quantité de produit au patient. La quantité délivrée dépend de la variation du volume dans le cylindre engendrée par le déplacement du piston dans le cylindre de la seringue. En utilisant la loi de Boyle-Mariotte qui énonce que PV= constante (à température constante), le médecin peut calculer la course du piston nécessaire pour délivrer une certaine quantité de produit. Le déplacement du piston engendre une augmentation de la pression dans l'enceinte jusqu'à que la dose correspondante soit expulsée de la poche.

Cependant, dans le dispositif décrit dans US4778451, la précision du dosage dépend du vérin : le piston est déplacé dans le cylindre de la seringue d'une course déterminée en fonction de la dose à délivrer. Ainsi, il faut tenir compte de la marge d'erreur du vérin pour doser le produit. Or cette marge d'erreur peut varier d'un dosage à l'autre, voir au cours d'un dosage, ce qui introduit un biais difficilement quantifiable dans le dosage du produit.

Par ailleurs, le dispositif selon de US4778451 comprend des joints d'étanchéité, par exemple au niveau du piston motorisé, ce qui peut être une source de contamination lorsque le dispositif est utilisé pour manipuler des produits différents.

Enfin, le dispositif selon US4778451 est encombrant notamment à cause de la présence du système de pompe motorisé.

Le document US6604908 décrit un dispositif de pompage volumétrique, comprenant une chambre de pompage et une chambre de contrôle séparées par une membrane flexible, permettant de faire varier le volume de la chambre de pompage. Le document US2013310743 décrit un tube flexible pincé entre deux doigts qui permettent le transit d'une matière à travers le tube. Le document US6213354 décrit un dispositif de dispense d'un produit sous forme de gouttelettes dans lequel la pression d'introduction du produit est déterminée en amont, en fonction de la taille des gouttelettes à obtenir. Le document EP1391734 décrit l'utilisation d'une seringue pour l'injection de produits dans un milieu réactionnel. Le document GB 1335797 décrit un pistolet injecteur permettant de créer une dépression pour aspirer la peau du patient sur l'aiguille de l'injecteur.

Il existe un besoin d'un dispositif de préparation d'une dose de quantité de matière qui facilite le dosage et permette de doser une quantité de matière plus précisément et de manière plus reproductible qu'avec les dispositifs existant.

### Bref résumé de l'invention

Un but de la présente invention est de proposer un dispositif ou un procédé exempt des limitations ou qui minimisent les limitations des dispositifs ou des procédés connus.

Selon l'invention, ce but est atteint notamment au moyen d'un dispositif pour préparer une dose de matière, le dispositif comprenant :
- un réservoir contenant la matière à doser, le réservoir étant en liaison fluidique avec une chambre pour doser la matière, la chambre étant en liaison fluidique avec un récipient destiné à recevoir la dose de matière, de sorte que la matière à doser transite par la chambre entre le réservoir et le récipient,
- le dispositif comprenant une source de vide pour établir une pression initiale Pi dans la chambre, et un capteur de pression pour mesurer la pression dans la chambre, l'entrée de matière dans la chambre induisant une variation de la pression dans la chambre de la pression initiale Pi à une pression finale Pf, la différence entre la pression initiale Pi et la pression finale Pf correspondant, à température constante, à la dose de matière,
   - le dispositif comprenant un module de commande connecté au capteur de pression,
      caractérisé en ce que ledit module contrôle la liaison fluidique entre la chambre et le réservoir, de façon à réguler l'introduction de matière dans la chambre, en fonction de la pression dans la chambre mesurée par le capteur de pression, où le module de commande limite l'introduction de matière dans la chambre à la quantité nécessaire pour faire passer la pression de la chambre de la pression initiale à la pression finale Pf, et où le module de commande contrôle l'entrée de matière pour ajuster la pression de la chambre à la pression finale.

Cette solution présente notamment l'avantage par rapport à l'art antérieur de fournir un dispositif basé sur la loi de Boyle Mariotte, où la matière est dosée en utilisant la variation de pression dans une chambre du dispositif. En effet, a température constante et à volume constant (ou calibré), l'introduction de matière la chambre fait varier la pression dans la chambre et cette variation est proportionnelle à la quantité de matière introduite dans la chambre. Ainsi, en connaissant la pression initiale Pi, il est possible de calculer une pression finale Pf, la différence entre la pression initiale Pi et la pression finale Pf correspondant, à la dose de matière. Réciproquement, en connaissant la pression initiale Pi à établir pour préparer une dose. Ainsi, lorsque la pression dans la chambre a atteint la pression finale Pf par introduction de matière, et que la pression dans la chambre est maintenue à la pression finale Pf, la chambre contient une dose de matière.

Dans le dispositif selon l'invention, la pression dans la chambre est mesurée par un capteur de pression relié à un module de commande. Le module de commande contrôle la liaison fluidique entre un réservoir, qui contient de la matière, et la chambre : ainsi, le module de commande régule l'entrée de matière dans la chambre en fonction de la pression mesurée par le capteur de pression. Le module de commande limite l'introduction de matière dans la chambre à la quantité nécessaire pour faire passer la pression de la chambre de la pression initiale Pi à la pression finale Pf. Le module de commande contrôle l'entrée de matière pour ajuster la pression de la chambre à la pression finale Pf. Avantageusement, le module de commande commande l'entrée de la matière ce qui permet de simplifier et de faciliter la préparation de la dose. Dans la présente invention, le module de commande effectue un contrôle continu de la pression dans la chambre au fur et à mesure de l'entrée de la matière (on parle de boucle fermée).Cela permet d'améliorer la précision de la quantité de matière dosée.

Les dispositifs de l'art antérieur ne décrivent pas de module de commande qui contrôle la liaison fluidique entre le réservoir et la chambre. Les dispositifs de l'art antérieur ne limitent pas l'introduction de matière dans la chambre à la dose recherchée. L'art antérieur ne décrit pas de module de commande qui régule l'entrée de matière en fonction de la variation de la pression dans la chambre.

Dans un mode de réalisation, le module de commande calcule la pression finale Pf à atteindre dans la chambre. Dans un mode de réalisation, le module de commande calcule la pression initiale Pi à établir dans la chambre. Ainsi, le dispositif est autonome, le calcul est intégré dans le module de commande, il n'y a pas besoin d'un module supplémentaire pour calculer la pression finale à atteindre Pf ou la pression initiale à établir Pi (pression finale Pf).

Selon un mode de réalisation, la liaison fluidique entre le réservoir et la chambre comprend une vanne d'entrée qui présente un temps de commutation t1 entre une position ouverte et une position fermée, le module de commande contrôle l'ouverture et la fermeture de ladite vanne, ledit module tenant compte dudit temps de commutation t1 pour que la chambre atteigne la pression finale Pf. Par exemple, le module de commande peut anticiper la fermeture de la vanne d'un temps t1, ce temps t1 correspondant au temps de commutation de la vanne, pour empêcher qu'une quantité supérieure à la dose n'entre dans la chambre, ce qui se traduirait par une pression dans la chambre supérieure à la pression Pf.

Dans un mode de réalisation, le module de commande tient compte des temps de commutation des vannes de vides ou des vannes de pression pour ajuster la pression dans la chambre. Dans un mode de réalisation, le module de commande tient compte des temps de commutation des vannes qui sont impliquées dans la préparation de la dose (autrement dit le dosage).

Dans un mode de réalisation, la liaison fluidique entre la chambre et le réservoir comprend un limiteur de débit, ledit limiteur étant soit manuel soit contrôlé par le module de commande. Le limiteur de débit permet de limiter le débit qui arrive à la vanne d'entrée de la chambre, ce qui facilite la fermeture de la vanne d'entrée et améliore ainsi la précision du dosage. Le limiteur permet ainsi de travailler sur toute la plage de dépression (par exemple de 0 à 1 bar absolu), tout en maintenant un débit limité à la vanne d'entrée pour garantir la précision de dosage.

Dans un mode de réalisation, la liaison fluidique entre la chambre et le récipient comprend une vanne de sortie, le module de commande contrôlant la vanne de sortie pour actionner le transfert de la dose dans le récipient

Selon un mode de réalisation, le dispositif comprend une source de pression reliée à la chambre, la source de pression étant soit manuelle soit contrôlée par le module de commande. La source de pression induit un flux de gaz qui qui a effet propulseur en favorisant la sortie de la dose de la chambre. La source de pression peut être contrôlée par le module de commande, de sorte que lorsque la dose est dans la chambre, le module de commande actionne la source de pression pour induire la sortie de la dose de la chambre, par exemple vers le récipient. Par exemple, le gaz peut être de l'air, de l'argon A, de l'azote N₂.

Selon un mode de réalisation adapté à la préparation de dose de matière corrosive. Avantageusement, la matière en transit dans le dispositif selon l'invention n'entre pas en contact avec des éléments qui serait sensibles avec des matières corrosives, par exemple des joints types o-ring connus pour se dégrader en présence de matières corrosives, ou toute autre dispositif d'étanchéité utilisant un élément en élastomère. Par exemple la source de vide (voir la source de pression) est isolée de la matière, les composants de la source de vide n'entrent pas en contact avec la matière.

Avantageusement, comme la matière ne transite pas par la source de vide, le nettoyage ou la stérilisation du dispositif est facilité puisque seuls les liaisons fluidiques, le réservoir et la chambre (voir le récipient) sont en contact avec la matière.

Dans un mode de réalisation la liaison fluidique entre le réservoir et la chambre comprend un conduit d'entrée reliant le réservoir et la chambre, le conduit d'entrée débouchant sur une vanne d'entrée qui contrôle l'entrée de la matière du réservoir vers la chambre.

Selon un mode de réalisation, la liaison fluidique entre la chambre et le récipient comprend un conduit de sortie reliant la chambre et le réservoir, le conduit de sortie comprenant une vanne de sortie de sortie qui contrôle la sortie de la matière du réservoir vers le récipient.

Dans un mode de réalisation, la liaison fluidique entre le réservoir et la chambre et/ou la liaison fluidique entre la chambre et le récipient comprend un module anti retour, ledit module anti retour étant soit manuel soit contrôlé par le module de commande, par exemple une bille flottante, un clapet ou une vanne.

Selon un mode de réalisation, le dispositif comprend une vanne à membrane, par exemple comme la vanne d'entrée et de sortie de la matière au niveau de la chambre. La membrane est composée d'une partie souple et épaisse recouverte d'un film fin de PTFE. Ce dernier est le seul élément en contact avec la matière. La mise en pression de la membrane ferme la vanne. La mise à l'atmosphère ou la mise sous vide ouvre la vanne.

Selon un mode réalisation, le dispositif comprend une combinaison de vannes et de clapets anti retour.

L'invention concerne également un procédé de préparation d'une dose de matière, le procédé comprenant les étapes suivantes :
- i) Fournir un dispositif de dosage comprenant
   o un réservoir contenant de la matière à doser, le réservoir étant en liaison fluidique avec une chambre pour doser la matière,
   ∘ une source de vide établir une pression initiale Pi dans la chambre, l'entrée de matière dans la chambre induisant une variation de la pression dans la chambre de la pression initiale Pi à une pression finale Pf, et un capteur de pression pour mesurer la pression dans la chambre,
- si la pression initiale Pi est prédéterminée,
   ∘ ii)a) Etablir la pression initiale Pi dans la chambre et mesurer ladite pression initiale Pi de la chambre avec le capteur de pression, la pression initiale Pi correspondant à la pression dans la chambre avant l'introduction de la matière;
   ∘ ii) b) Déterminer la dose de matière à préparer;
   ∘ ii) c) Calculer la pression finale Pf à atteindre dans la chambre pour préparer ladite dose ;
- ou, si la pression finale Pf est prédéterminée,
   ∘ ii)d) Déterminer la dose de matière à préparer;
   ∘ ii)e) Calculer la pression initiale Pi à établir dans la chambre pour préparer la dose en fonction de la pression finale Pf à atteindre dans la chambre, la pression finale Pf correspondant à la pression dans la chambre après l'introduction de la dose;
   ∘ ii)f) Etablir la pression initiale Pi dans la chambre et mesurer ladite pression initiale Pi de la chambre;
- iii) Transférer de la matière à doser du réservoir vers dans la chambre via la liaison fluidique jusqu'à ce que la pression de la chambre atteigne la pression finale Pf de façon à introduire dans la chambre la dose de matière.

Le procédé selon l'invention utilise la loi de Boyle Mariotte pour déterminer la variation de pression nécessaire dans la chambre pour faire entrer une dose de matière dans la chambre. En connaissant la pression initiale Pi ou la pression finale Pf et la masse de la dose à préparer, il est possible de déterminer la pression à atteindre dans la chambre pour que la chambre contienne la dose de matière recherchée.

Le procédé selon l'invention comprend deux alternatives :

Dans la première alternative, la pression initiale est prédéterminée, autrement dit connue ou fixée à l'avance, avant de réaliser le dosage. Une pression initiale Pi est établie dans la chambre par la source de vide, puis de la matière est introduite dans la chambre jusqu'à ce que la pression dans la chambre atteigne une pression finale Pf, la différence entre la pression initiale Pi et la pression finale Pf correspondant, à température constante, à la dose de matière à préparer (étape i), ii) a, ii) b), ii)c)). Dans ce mode de réalisation, la pression initiale Pi est fixée ou déterminée et la pression finale Pf à atteindre, pour préparer la dose, est calculée en fonction de cette pression initiale Pi fixée. Par exemple la pression initiale Pi est fixée à 0 mbar absolu, ou proche de 0 mbar absolu.

Dans la deuxième alternative, la pression finale Pf est prédéterminée, autrement dit connue ou fixée. La pression initiale Pi à établir est calculée en fonction de la pression finale Pf, de façon à ce que la chambre contienne la dose lorsque la pression dans la chambre est passée de la pression initiale Pi à la pression finale Pf (étape i), ii) d, ii)e), ii)f)). Ce mode de réalisation est avantageux lorsqu'on veut atteindre une pression finale déterminée, par exemple entre 100 et 500mbar absolu.

Avantageusement, selon la présente invention, il y a un contrôle continu de la pression durant l'entrée de la matière dans la chambre, au fur et à mesure de l'entrée de matière (on parle aussi de boucle fermée), pour veiller à ce que la pression dans la chambre soit maintenue entre la pression initiale Pi et la pression finale Pf, de façon à arrêter l'entrée de matière lorsque la pression dans chambre a atteint la pression finale Pf. Ce contrôle est assuré par le module de commande.

Selon un mode de réalisation, le procédé comprenant l'étape suivante :
- Calibrer la chambre pour déterminer la variation du volume de la chambre en fonction de la variation de la pression dans chambre.
   Il est possible que le volume de la chambre varie en fonction de la pression à l'intérieur de la chambre, cela dépend notamment du matériau de la chambre et de la pression utilisée. Par exemple, la chambre à un volume V1 à une pression P1, mais lors de la mise sous vide de la chambre a une pression P2 (P2<P1) les parois de la chambre se rapprochent et le volume de la chambre diminue à un volume V2 (V2 < V1). Par exemple, on observe ce phénomène en utilisant une chambre en tube de plastique souple, où les parois ont tendance à se rapprocher l'une vers l'autre sous vide. Cette variation du volume en fonction de la pression introduit un biais dans le dosage dont on peut tenir compte pour améliorer la précision du dosage. Pour cela, dans ce mode réalisation, le procédé comprend une étape de calibration pour déterminer le volume de la chambre en fonction de la pression à l'intérieur de la chambre. Cette calibration sera prise en compte lors du calcul de la pression finale Pf à atteindre, ou de la pression initiale Pi à établir. De préférence, la calibration est faite uniquement une fois à la mise en service. Elle peut être faite de manière automatique en lançant « une calibration » ou de manière manuelle.

Dans un mode de réalisation, le procédé comprend :
- Calculer la pression finale Pf à atteindre dans la chambre pour préparer la dose, en fonction de la densité de la matière à doser, de la pression initiale Pi, du volume Vi de la chambre; ou
- calculer la pression initiale Pi à établir dans la chambre pour préparer la dose, en fonction de la densité de la matière à doser, de la pression finale Pf, du volume Vi de la chambre.

Lorsque le volume à prélever n'est pas connu, il est possible de calculer la pression finale Pf à atteindre ou la pression initiale Pi à établir en fonction de la densité de la matière, de la pression initiale Pi ou de la pression finale Pf et du volume Vi de la chambre.

Selon un mode de réalisation, la chambre est en liaison fluidique avec un récipient destiné à recevoir la dose de matière, de sorte que la matière à doser transite par la chambre entre le réservoir et le récipient, le procédé comprenant l'étape suivante :
- Transférer la dose de matière depuis la chambre vers le récipient.
   Les récipients peuvent être de nature très différente : flacons, bouteilles, moules, bidons, réacteurs, bains, seringues, blisters, etc.

Dans un mode de réalisation, la liaison fluidique entre le réservoir et la chambre comprend une vanne d'entrée qui présente un temps de commutation t1 entre une position ouverte et une position fermée, l'étape v) comprenant :
- adapter l'actionnement de la vanne d'entrée en fonction du temps de commutation t1 pour introduire de la matière à doser dans la chambre jusqu'à ce que la pression de la chambre atteigne la pression finale Pf.

Ainsi, il est possible d'anticiper la fermeture de la vanne d'entrée en fonction de ce temps de commutation pour éviter qu'il y ait un excès de matière entrant dans la chambre, ce qui induirait un biais dans le dosage.

Selon un mode de réalisation, le dispositif comprenant une source de pression reliée à la chambre, par exemple un compresseur, le procédé comprenant l'étape suivante :
- Actionner la source de pression pour fournir un flux de gaz, par exemple de l'air, dans la chambre et transférer la dose depuis la chambre vers le récipient.
   La libération de la dose peut se faire soit en utilisant la gravité en laissant la matière sortir de la chambre, par exemple vers le récipient. Alternativement, on peut accélérer la sortie de la matière en utilisant une source de compression pour pousser la matière vers la sortie de la chambre.

Selon un mode de réalisation, le procédé comprend :
- Etablir une pression initiale Pi dans la chambre en ajustant la pression dans la chambre en plusieurs étapes pour atteindre la pression initiale Pi, chaque étape étant espacé d'au moins 1 seconde; et/ou
- Transférer de la matière à doser du réservoir vers dans la chambre, le transfert étant réalisé par palier, chaque palier étant séparé d'au moins 1 seconde.
   L'établissement de la pression initiale Pi dans la chambre peut se faire de façon continue, sans étape, ou de façon discontinue en plusieurs étapes. Dans le mode discontinu, on utilise plusieurs paliers successifs pour atteindre la pression initiale, chaque étape est espacée d'au moins une seconde. Par exemple, la pression initiale peut être atteinte avec un premier palier à 95% de la pression initiale, puis, après un temps de latence de 1 seconde, la pression initiale est atteinte. Le temps de latence peut être aussi être de plusieurs secondes, ou dizaine de secondes. Le temps entre chaque étape permet de stabiliser la pression dans chambre, pour améliorer la précision de la mesure de la pression dans la chambre.
   L'entrée de matière dans la chambre peut se faire de façon continue, sans palier, ou de façon discontinue en respectant des paliers. Dans le mode discontinu, la matière entre dans la chambre en plusieurs paliers, une fraction de matière par palier. Par exemple, 95 % de la matière entre dans la chambre en limitant la pression la chambre à 95 % de la pression finale Pf. Puis, après un temps de stabilisation, par exemple de 1 seconde, le reste de la matière entre dans la chambre pour atteindre la pression finale Pf. Le temps de stabilisation permet d'améliorer la précision sur la masse de la dose. L'utilisation de paliers permet d'améliorer la précision pour atteindre la pression finale Pf.

Selon un mode de réalisation, la chambre comprend un système anti reflux, pour bloquer la sortie de la matière vers le récipient. Par exemple, le système anti reflux est une bille flottante. L'utilité est d'éviter d'introduire de l'air de propulsion dans la liaison fluidique entre la chambre et le récipient. En effet, il est en règle générale pas souhaitable d'avoir de l'air qui s'expulse lorsque la matière a fini d'être transférée dans le récipient.

Dans un mode de réalisation, le dispositif ou le procédé permet une précision de dosage de l'ordre de 0,5 % pour des doses correspondants à des volumes entre 1 cc et 10000cc. Si les paramètres du dispositif sont constants d'un dosage au suivant (par exemple volume de la chambre etc...), alors la précision de dosage est la précision de mesure du capteur de pression.

Selon un mode de réalisation, le dispositif ou le procédé peut être utilisé pour pomper un liquide d'un réservoir vers un récipient, comme une pompe. La chambre est mise sous vide à une pression déterminée arbitrairement, puis le liquide est aspiré dans la chambre jusqu'à ce que la chambre soit à pression ambiante ou jusqu'à ce que le réservoir soit vide, et transféré dans le récipient. Dans ce mode de fonctionnement, il est possible de se passer du capteur de pression. Le système de contrôle commande le déroulement de la séquence par des temporisations.

Selon un mode de réalisation, le module de commande présente des temps de cycle (autrement dit des temps de rafraichissements des données qui parviennent au module de commande) de l'ordre de la milliseconde, par exemple 20 ms. Mais l'invention et également compatible avec des module de commande présentant des temps de cycle plus court, dit « en temps réel », par exemple de l'ordre de la micro seconde.

Avantageusement, le dispositif selon l'invention peut au moins partiellement être placé dans un environnement ATEX car il présente un nombre minimum de source de chaleur (autrement dit source d'ignition), voire aucune source de chaleur. Typiquement, les dispositifs de dosage selon l'art antérieur comprennent un moteur : les moteurs ont des surfaces chaudes qui peuvent créer des étincelles si le moteur est en court-circuit et dans le pire des cas s'enflammer.

Par exemple, le module de contrôle peut être composé d'éléments ATEX ou être déplacé hors zone de risque.

Dans un mode de réalisation, le procédé comprenant une étape de programmation d'un module de commande pour exécuter de façon automatique au moins l'une des étapes suivantes :
- si la pression initiale Pi est prédéterminée,
   ∘ ii)a) Etablir la pression initiale Pi dans la chambre (3, 103, 203) et mesurer ladite pression initiale Pi de la chambre (3, 103, 203) avec le capteur de pression (20, 120, 220), la pression initiale Pi correspondant à la pression dans la chambre (3, 103, 203) avant l'introduction de la matière;
   ∘ ii) b) Déterminer la dose de matière à préparer;
   ∘ ii) c) Calculer la pression finale Pf à atteindre dans la chambre (3, 103, 203) pour préparer ladite dose;
- ou, si la pression finale Pf est prédéterminée,
   ∘ ii)d) Déterminer la dose de matière à préparer;
   ∘ ii)e) Calculer la pression initiale Pi à établir dans la chambre (3, 103, 203) pour préparer la dose en fonction de la pression finale Pf à atteindre dans la chambre (3, 103, 203), la pression finale Pf correspondant à la pression dans la chambre après l'introduction de la dose;
   ∘ ii)f) Etablir la pression initiale Pi dans la chambre (3, 103, 203) et mesurer ladite pression initiale Pi de la chambre (3, 103, 203) ;
- iii) Transférer de la matière à doser du réservoir (2, 102, 202) vers la chambre (3, 103, 203) via la liaison fluidique jusqu'à ce que la pression de la chambre (3, 103, 203) atteigne la pression finale Pf de façon à introduire dans la chambre (3, 103, 203) la dose de matière.
- Transférer de la matière à doser du réservoir vers dans la chambre via la liaison fluidique jusqu'à ce que la pression de la chambre atteigne la pression finale Pf de façon à introduire dans la chambre la dose de matière ;
- Calibrer la chambre pour déterminer la variation du volume de la chambre en fonction de la variation de la pression dans chambre ;
- Déterminer une pression finale Pf à atteindre dans la chambre pour préparer la dose en fonction de la densité de la matière à doser, de la pression initiale Pi et du volume Vi de la chambre;
- Transférer la dose de matière depuis la chambre vers le récipient ;
- Adapter l'actionnement de la vanne d'entrée en fonction du temps de commutation t1 pour introduire de la matière à doser dans la chambre jusqu'à ce que la pression de la chambre atteigne la pression finale Pf ;
- Actionner la source de pression pour fournir un flux de gaz, par exemple de l'air, dans la chambre et transférer la dose depuis la chambre vers le récipient.
- Etablir une pression initiale Pi dans la chambre en ajustant la pression dans la chambre en plusieurs étapes pour atteindre la pression initiale, chaque étape étant espacée d'au moins 1 seconde; et/ou
- Transférer de la matière à doser du réservoir vers dans la chambre, le transfert étant réalisé par palier, chaque palier étant séparé d'au moins 1 seconde.

Ainsi, dans ce mode de réalisation il est possible d'exécuter de façon automatique toutes ou un certain nombre d'étapes du procédé. La manutention qui va autour du dosage, comme disposer le récipient vide à la bonne place, changer ou re-remplir le réservoir lorsqu'il est vide, déplacer le récipient plein ou lui mettre un couvercle etc... peut être fait de manière manuelle ou automatique.

Dans un mode de réalisation, le procédé comprenant une étape de programmation d'un module de commande pour exécuter de façon automatique au moins les étapes suivantes :
- La pression initiale Pi est prédéterminée :
   o Etablir la pression initiale Pi prédéterminée dans la chambre et mesurer ladite pression initiale Pi de la chambre avec le capteur de pression, la pression initiale Pi correspondant à la pression dans la chambre avant l'introduction de la matière;
   o Déterminer la dose de matière à préparer : le volume à prélever est connu ou calculé;
   o Calculer la pression finale Pf à atteindre dans la chambre pour préparer ladite dose ;
- Transférer de la matière à doser du réservoir vers la chambre via la liaison fluidique jusqu'à ce que la pression de la chambre atteigne la pression finale Pf de façon à introduire dans la chambre la dose de matière. Lors du transfert, autrement dit le remplissage, il y a une mesure continue de la pression dans la chambre.
- Lorsque la valeur de la pression finale est atteinte, alors la vanne d'entrée est fermée.
- Transférer la dose de matière depuis la chambre vers le récipient;

Ce mode de réalisation permet d'atteindre les meilleures performances en termes de temps de dosage. Dans ce cas-là, la pression initiale est fixe et dépend de la source de vide, typiquement proche de 0 mbar absolu. Ce mode de réalisation permet d'obtenir des dosages avec une précision standard de 2 à 3 %.

Dans un mode de réalisation, le procédé comprenant une étape de programmation d'un module de commande pour exécuter de façon automatique au moins les étapes suivantes :
- La pression finale Pf est prédéterminée
   o Déterminer la dose de matière à préparer; le volume à prélever est connu ou calculé;
   ∘ Calculer la pression initiale Pi à établir dans la chambre pour préparer la dose en fonction de la pression finale Pf à atteindre dans la chambre, la pression finale Pf correspondant à la pression dans la chambre après l'introduction de la dose;
   o Etablir la pression initiale Pi dans la chambre et mesurer ladite pression initiale Pi de la chambre;
- Transférer de la matière à doser du réservoir vers la chambre via la liaison fluidique jusqu'à ce que la pression de la chambre atteigne la pression finale Pf de façon à introduire dans la chambre la dose de matière. Lors du transfert, autrement dit le remplissage, il y a une mesure continue de la pression dans la chambre.
- Lorsque la valeur de la pression finale Pf prédéterminée est atteinte, alors la vanne d'entrée est fermée.
- Transférer la dose de matière depuis la chambre vers le récipient;

Ce mode de réalisation permet d'atteindre les meilleures performances en termes de précision de dosage. Dans ce cas-là, la pression finale est fixe et est déterminée de manière à avoir un bon compromis temps/précision. Typiquement la valeur de pression finale est arbitrairement choisie entre 100 et 500mbar absolu. Ce mode de réalisation permet d'atteindre une précision de dosage de 1 % à 2 %.

Dans un mode de réalisation, le procédé comprenant une étape de programmation d'un module de commande pour exécuter de façon automatique au moins les étapes suivantes :
- La pression finale Pf est prédéterminée
   o Déterminer la dose de matière à préparer; le volume à prélever est connu ou calculé;
   ∘ Calculer la pression initiale Pi à établir dans la chambre pour préparer la dose en fonction de la pression finale Pf à atteindre dans la chambre, la pression finale Pf correspondant à la pression dans la chambre après l'introduction de la dose;
   o Etablir la pression initiale Pi dans la chambre la pression initiale Pi étant établi en ajustant la pression dans la chambre en plusieurs étapes pour atteindre la pression initiale, chaque étape étant espacé d'au moins 1 seconde, pour que la valeur de pression se stabilise;
   o Mesurer ladite pression initiale Pi de la chambre; répéter l'étape précédente si la pression mesurée est différente de la pression initiale Pi calculée;
- Transférer de la matière à doser du réservoir vers la chambre via la liaison fluidique jusqu'à ce que la pression de la chambre atteigne la pression finale Pf de façon à introduire dans la chambre la dose de matière. Lors du transfert, autrement dit le remplissage, il y a une mesure continue de la pression dans la chambre ; Le transfert est réalisé par palier, chaque palier étant séparé d'au moins 1 seconde.
- Lorsque la valeur de la pression finale Pf prédéterminée est atteinte, alors la vanne d'entrée est fermée, l'actionnement de la vanne est adapté en fonction du temps de commutation de la vanne ;
- Mesure de la pression dans la chambre ; si la valeur de pression est différente de la pression finale Pf, alors l'étape précédente est répétée ;
- Lorsque la valeur de la pression finale Pf prédéterminée est atteinte, alors la vanne d'entrée est fermée ;
- Transférer la dose de matière depuis la chambre vers le récipient;

Ce mode de réalisation permet de prendre en compte les retards dû à la fermeture de vannes, autrement dit les temps de commutation. Ce mode de réalisation intègre aussi les étapes de mise sous pression et le remplissage par palier, pour tenir compte des temps de stabilisation de la mesure. Ce mode de réalisation permet d'atteindre une précision de dosage inférieure à 1%.

Dans un mode de réalisation, le procédé comprenant une étape de programmation d'un module de commande pour exécuter de façon automatique au moins les étapes suivantes :
- Calibrer la chambre pour déterminer la variation du volume de la chambre en fonction de la variation de la pression dans chambre ;
- La pression finale Pf est prédéterminée
   ∘ Déterminer la dose de matière à préparer; le volume à prélever est connu ou calculé;
   ∘ Calculer la pression initiale Pi à établir dans la chambre pour préparer la dose en fonction de la pression finale Pf à atteindre dans la chambre, la pression finale Pf correspondant à la pression dans la chambre après l'introduction de la dose;
   ∘ Etablir la pression initiale Pi dans la chambre la pression initiale Pi étant établi en ajustant la pression dans la chambre en plusieurs étapes pour atteindre la pression initiale, chaque étape étant espacé d'au moins 1 seconde, pour que la valeur de pression se stabilise;
   ∘ Mesurer ladite pression initiale Pi de la chambre; répéter l'étape précédente si la pression mesurée est différente de la pression initiale Pi calculée;
- Transférer de la matière à doser du réservoir vers la chambre via la liaison fluidique jusqu'à ce que la pression de la chambre atteigne la pression finale Pf de façon à introduire dans la chambre la dose de matière. Lors du transfert, autrement dit le remplissage, il y a une mesure continue de la pression dans la chambre ; Le transfert est réalisé par palier, chaque palier étant séparé d'au moins 1 seconde.
- Lorsque la valeur de la pression finale Pf prédéterminée est atteinte, alors la vanne d'entrée est fermée, l'actionnement de la vanne est adapté en fonction du temps de commutation de la vanne ;
- Mesure de la pression dans la chambre ; si la valeur de pression est différente de la pression finale Pf, alors l'étape précédente est répétée ;
- Lorsque la valeur de la pression finale Pf prédéterminée est atteinte, alors la vanne d'entrée est fermée ;
- Transférer la dose de matière depuis la chambre vers le récipient;
   Ce mode de réalisation permet d'atteindre des précisions extrêmes, très inférieure à 1%, grâce une étape de calibration pour tenir compte des imperfections du dispositif, par exemple la variation du volume de la chambre en fonction de la pression (ou encore : volume de chambre suivant tolérance de fabrication ; Imprécision du capteur de pression, etc.). Une procédure de calibration est réalisée à la mise en service, et peut ensuite être réalisée à intervalle régulier (typiquement sur une base annuelle) si besoin. Il est avantageux de vérifier la calibration, par exemple à chaque début de campagne de production, en prenant quelques échantillons pour les peser sur une balance séparée.

Par exemple, la procédure de calibration comprend typiquement les étapes suivantes :
- Doser quelques échantillons qui correspondent au minium, environ à la moitié et au maximum dosable (par exemple (100g, 500g, 1kg)) ;
- peser individuellement sur une balance séparée les échantillons dosés à l'étape précédente : ces valeurs réelles mesurées sur une balance séparée sont ensuite saisies dans le module de commande qui va ainsi calculer des facteurs de corrections pour prendre en compte les imprécisions du système.
- Exemple : La dose demandée est de 500g (consigne) ; l'échantillon dosé est pesé et sur la balance, on a pesé 505g (réalisé). On saisit la valeur réellement dosée de 505g pour une consigne de 500g dans le système de commande qui va calculer la valeur est 1% trop haute (505/500) et va ensuite compenser cette erreur en dosant 495.05 g lorsque la consigne est demandée. Ceci fera que la dose réellement réalisée sera de 495.05+1% = 500g.

La pression initiale Pi est la pression dans la chambre avant l'introduction de matière. La source de vide établi la pression initiale Pi. La pression finale Pf est supérieure à la pression initiale Pi.

A la pression initiale, la chambre est sous vide, c'est-à-dire à une pression inférieure à la pression atmosphérique, de sorte qu'une partie au moins de la matière est aspirée dans la chambre lors de l'introduction de la matière. Par exemple :
- Pi = -0.9 bar; P cal = -0.2 bar

Il est aussi possible que la pression initiale Pi soit égale ou supérieure à la pression atmosphérique. Il est alors nécessaire d'entrainer la matière dans la chambre par une force supplémentaire, par exemple la gravité ou en utilisant une pompe péristaltique. Par exemple :
- Pi = P atm = 1.01325 bar; Pfalc = 1.5 bar

Dans un mode de réalisation, la pression initiale Pi est inférieure à Pf, avec Pi inférieure à la pression atmosphérique P atm, et Pf inférieure ou égale à P atm :
Pi<Pf, avec Pi < Patm et Pf ≤ P atm.
Dans ce cas, il y a une dépression dans la chambre, la matière est aspirée dans la chambre jusqu'à ce que la pression de la chambre atteigne la pression finale Pf. Ce mode de fonctionnement est avantageux car le vide fourni l'énergie nécessaire au transport de la matière.

Dans un autre mode de réalisation, la pression initiale Pi est inférieure à Pf, avec Pi inférieure ou égale à la pression atmosphérique P atm, et Pf supérieure à la pression atmosphérique Patm :
Pi<Pf, avec Pi ≤ P atm et Pf> P atm.
Dans ce cas, la pression finale Pf est supérieur à la pression atmosphérique, de sorte qu'il faut exercer une pression sur la matière dans le réservoir pour faire entrer la matière dans la chambre. Par exemple, le réservoir peut être positionné au-dessus de la chambre pour utiliser la force de gravité. L'avantage de ce mode de réalisation est sa simplicité car il n'y a pas besoin de pompe à vide. Alternativement, on peut brancher une pompe péristaltique sur le réservoir.

L'invention concerne également un système pour préparer une composition, la composition comprenant plusieurs doses de matière différentes, le système comprenant :
- Une pluralité de réservoir et une pluralité de chambre, chaque réservoir étant en liaison fluidique avec une chambre, lesdites chambres étant en liaison fluidique avec au moins un récipient destiné à recevoir les doses de matière de chaque chambre, de sorte que chaque dose transite entre un des réservoirs et le récipient;
- Au moins une source de vide pour faire varier la pression dans lesdites chambres d'une pression initiale Pi à une pression finale Pf, et au moins un capteur de pression pour mesurer la pression dans les chambres, la différence entre la pression initiale Pi et la pression finale Pf correspondant, dans chaque chambre, à température constante, à la dose de matière à préparer par chaque chambre,
caractérisé en ce que le système comprend un module de commande connecté au capteur de pression, ledit module contrôlant les liaisons fluidiques entre les chambres et les réservoirs de façon à réguler l'introduction de matière dans les chambres en fonction de la pression mesurée par le capteur de pression dans la chambre jusqu'à ce que la pression de la chambre atteigne la pression finale Pf.

Le système peut ainsi comprendre plusieurs ensembles « réservoir-chambre » en parallèle, chaque ensemble préparant une dose de matière distincte, puis les doses sont collectées dans un récipient pour former la composition. Le système peut comprendre une source de vide unique et éventuellement une source de pression unique, les sources de vide et de pression étant partagées par tous les ensembles. Alternativement, le système peut comprendre plusieurs sources de vide et éventuellement de pression, par exemple une source de vide et éventuellement une source de pression par ensemble.

Les termes « liaison fluidique », par exemple entre le réservoir et la chambre, définissent les éléments qui permettent de relier une pièce à une autre, par exemple le réservoir à la chambre, pour permettre le transfert d'un fluide. Par exemple, une liaison fluidique peut comprendre des tuyaux, des raccords, des vannes, des robinets, etc...

Les termes « module de commande » définissent un composant qui permet de gouverner le fonctionnement de certains composants, par exemple l'ouverture et la fermeture d'une vanne. Le module de commande permet aussi de coordonner l'action entre plusieurs composants, par exemple fermer une vanne lorsqu'un capteur de pression détecte que la pression dans la chambre a atteint une valeur seuil. Le module de commande peut comprendre un logiciel de commande, des composants électroniques, etc...Par exemple le module de commande peut être un automate.

Le terme « matière » défini une substance constituée de différents éléments, atomes, molécules ou ions. La matière utilisable dans la présente invention peut se présenter selon une large gamme de fluides, par exemple liquides, pâtes, suspensions, émulsions, etc. Dans la présente invention, la matière utilisable est capable d'être transférer d'un composant à un autre, par exemple du réservoir vers la chambre, et dont on peut déterminer la densité. La matière est de préférence un liquide ou un fluide, par exemple une solution aqueuse ou organique.

Dans la présente invention, la chambre est un composant qui peut recevoir de la matière et dont on peut faire varier la pression interne. Par exemple, la chambre peut être un cylindre avec une section déterminée, , une portion d'un tube en polymère plastique ou une chambre à base rectangulaire. La chambre peut être faite en différents matériaux, par exemple en acier, en polypropylène, en PTFE, PEEK, Hastelloy, ou en matériaux déformable sous la pression par exemple en silicones, notamment des tuyaux en silicones.. En particulier, le matériau de la chambre peut être adapté en fonction des propriétés de la matière à doser.

Dans la présente invention, le capteur de pression peut être un capteur de pression absolu ou relatif. Le capteur de pression relatif prend sa référence sur la pression ambiante. Le capteur de pression absolue s'affranchi de la variabilité de la pression ambiante, mais ces capteurs sont moins courants sur le marché. En fonction de l'utilisation (précision requise, conditions environnementales du dosage), il est possible d'utiliser l'un ou l'autre ou une combinaison de ces capteurs dans la présente invention.

Dans la présente invention, la source de vide peut être une pompe à palette lubrifiée, une pompe à anneau liquide, un venturi ou tout autre système capable de générer une dépression. Alternativement, la source de vide peut comprendre une pompe qui met sous vide une cuve de vide, la cuve de vide étant en liaison fluidique avec la chambre pour mettre la chambre à la pression initiale Pi recherchée.

Les modes de réalisations décrits pour le dispositif selon l'invention s'appliquent également au procédé et au système selon l'invention, mutatis mutandis.

### Brève description des figures

Des exemples de mise en œuvre de l'invention sont indiqués dans la description illustrée par les figures annexées dans lesquelles :
La figure 1 illustre l'invention selon premier mode de réalisation ;
La figure 2 illustre l'invention selon un deuxième mode de réalisation;
La figure 3 illustre l'invention selon un troisième de réalisation; Exemple(s) de mode de réalisation de l'invention

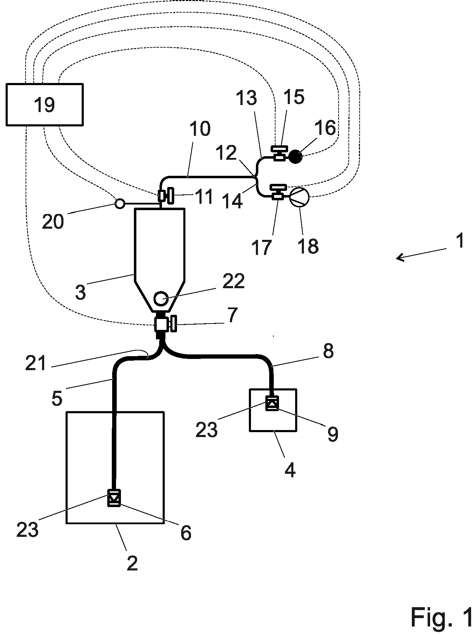

Des exemples de modes de réalisation sont représentés sur les figures 1 à 3, mais l'invention ne se limite pas à ces modes de réalisation.

La figure 1 représente un dispositif 1 selon un premier mode de réalisation. Le dispositif 1 comprend un réservoir 2 comprenant de la matière, ici un liquide, une chambre 3 et un récipient 4. La liaison fluidique entre le réservoir 2 et la chambre 3 comprend un conduit d'entrée 5 avec une vanne d'aspiration 6 à l'extrémité dans le réservoir 2, et une vanne d'entrée 7 à proximité de la chambre 3. La liaison fluidique entre la chambre 3 et le récipient 4 comprend un conduit de sortie 8 avec la vanne d'entrée 7 à proximité de la chambre 3 et une vanne de sortie 9. La vanne d'entrée 7 est une vanne trois voies, avec une voie pour le conduit d'entrée 5, une voie pour la chambre 3, une voie pour le conduit de sortie 9.

La chambre 3 est connectée à un conduit d'air 10, le conduit d'air 10 comprenant une vanne d'air 11 dont l'ouverture et la fermeture régule le flux d'entrée d'air dans la chambre. Dans cet exemple, il s'agit d'air mais l'air peut être remplacé par un gaz neutre comme de l'azote N₂ ou de l'argon Ar. Le conduit d'air 10 débouche sur un raccord 12 deux voies : une voie pour un conduit de vide 13 et une voie pour un conduit de pression 14.

Le conduit de vide 13 comprend une vanne de vide 15 et débouche sur une source de vide 16, ici une pompe à palette. Lorsque la source de vide 16 est activée, la vanne de vide 15 et la vanne d'air 11 ouvertes et la vanne d'entrée 7 fermée, alors la source de vide 16 permet de diminuer la pression dans la chambre 3.

Le conduit de pression 14 comprend une vanne de pression 17 et débouche sur une source de pression 18, ici un compresseur. Lorsque la source de pression 18 est activée, la vanne de pression 17 et la vanne d'air 11 ouvertes et la vanne d'entrée 7 fermée, alors la source de pression 18 permet d'augmenter la pression dans la chambre. Si la vanne d'entrée 7 est ouverte, alors la source de pression 18 accélère la sortie de la matière contenue dans la chambre 3 vers le récipient 4.

Le dispositif 1 comprend un module de commande 19. Le module de commande 19 est connecté à un capteur de pression 20 qui mesure la pression dans la chambre 3. Dans ce mode de réalisation, le module de commande contrôle également la vanne d'entrée 7, la vanne d'air 11, la source de vide 16 et la vanne de vide 17, la source de pression 18 et la vanne de pression 17.

Dans ce premier mode de réalisation, le dispositif comprend un limiteur de débit 21 sur le conduit d'entrée 5. La chambre 3 comprend une bille flottante 22 qui peut boucher l'ouverture où débouche le conduit d'entrée 5 dans la chambre 3. La vanne d'aspiration 6 et la vanne de sortie 9 comprennent chacune un clapet anti retour 23.

Le procédé pour préparer une dose matière en utilisant le dispositif selon ce premier mode de réalisation comprend les étapes suivantes :
- Ouvrir la vanne de vide 13 et la vanne d'air 10 pour établir une pression initiale Pi dans la chambre 3 ;
- Le capteur de pression mesure la pression initiale et transmet la mesure au module de commande ;
- Déterminer la dose de matière à préparer;
- Le module de commande 19 calcule la pression finale Pf à atteindre dans la chambre 3 pour préparer la dose de matière en fonction de la pression initiale mesurée, le volume de la chambre, la masse de la dose à préparer, la densité de la matière,
- Le module de commande 19 contrôle la vanne d'aspiration 6 et la vanne d'entrée 7 pour limiter l'entrée de matière dans la chambre 3 à la dose, c'est-à-dire la quantité nécessaire pour atteindre la pression finale Pf, en tenant compte des temps de commutation de la vanne d'aspiration 6 et de la vanne d'entrée 7 ;
- Une fois que la dose est dans la chambre 3 et que la vanne d'aspiration 6 et la vanne d'entrée 7 (voie conduit d'entrée) sont fermée, le module de commande 19 désactive la source de vide 16, ferme la vanne de vide 15 ;
- Le module de commande 19 ouvre la vanne d'entrée 7 (voie récipient), et la vanne de pression 18, puis active la source de pression 18 pour accélérer le transfert de la dose vers le récipient 4;
- Lorsque la totalité de la dose est sortie de la chambre 3, la bille flottante 22 bouche la sortie de la chambre 3 pour empêcher l'entrée du flux d'air (de la source de pression) vers le récipient 4; le module de commande 19 désactive la source de pression 18 .

Dans ce mode de réalisation, , la pression initiale Pi est inférieure à Pf, avec Pi inférieure à la pression atmosphérique P atm, et Pf inférieure à Patm :
Pi<Pf, avec Pi < Patm et Pf < P atm.

L'enchainement de ces étapes définit un cycle qui permet de préparer une dose. Dans ce mode de réalisation, le module de commande est programmé pour exécuter les étapes du cycle de façon automatique pour préparer et transférer un nombre déterminé de dose vers le récipient. L'utilisateur entre la masse de la dose, la densité de la matière, et le nombre de dose (égal au nombre de cycle). Le module de commande calcule la pression finale Pf à atteindre pour préparer la dose, et répète le cycle autant de fois que nécessaire. A la fin du cycle, l'utilisateur peut programmer un nouveau cycle.

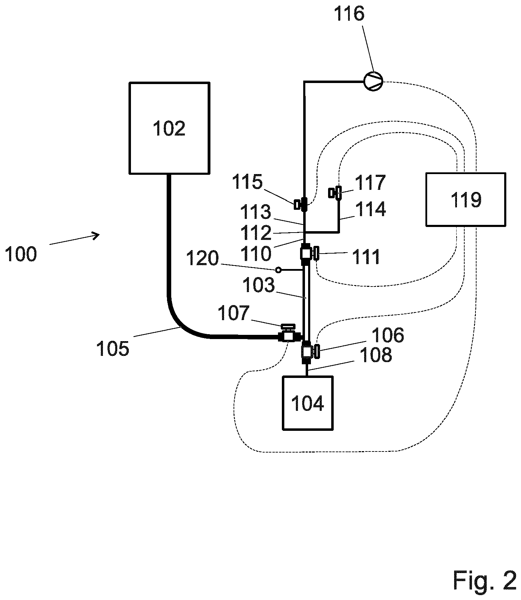

La figure 2 représente un dispositif 100 selon un deuxième mode de réalisation. Le dispositif 100 comprend un réservoir 102 comprenant de la matière, ici un liquide, une chambre 103 et un récipient 104. La liaison fluidique entre le réservoir 103 et la chambre 104 comprend un conduit d'entrée 105 et une vanne d'entrée 107 à proximité de la chambre 103. La liaison fluidique entre la chambre 3 et le récipient comprend un conduit de sortie 108 une vanne de récipient 106.

La chambre 103 est connectée à un conduit d'air 110, le conduit d'air 110 comprenant une vanne d'air 11 dont l'ouverture ou la fermeture régule le flux d'entrée d'air dans la chambre 103. Le conduit d'air 110 débouche sur un raccord 112 deux voies : une voie pour un conduit de vide 113 et une voie pour un conduit de pression 114.

Le conduit de vide 113 comprend une vanne de vide 115 et débouche sur une source de vide 116 pour diminuer la pression dans la chambre 103 comme décrit pour le premier mode de réalisation.

Le conduit de pression 114 comprend une vanne de pression 117. A la différence du premier mode de réalisation, le dispositif 100 selon le deuxième mode de réalisation ne comprend pas de source de pression. La vanne de pression 117 débouche sur l'environnement extérieur, où la pression est la pression atmosphérique. Alternativement, il est possible de brancher une réserve de gaz neutre sur la vanne de pression 120 pour les matières sensibles à l'air.

Le dispositif 100 comprend un module de commande 119. Le module de commande 119 est connecté à un capteur de pression 120 qui mesure la pression dans la chambre 3. Dans ce mode de réalisation, le module de commande contrôle également la vanne d'entrée 107, la vanne du récipient 106, la source de vide 116 et la vanne de vide 115, la vanne de pression 117.

Dans ce mode de réalisation, la pression initiale Pi est inférieure à Pf, avec Pi inférieure à la pression atmosphérique P atm, mais la pression finale Pf est supérieure à la pression atmosphérique Patm :
Pi<Pf, Pi < Patm avec Pf > P atm.
Pour que la chambre atteigne une pression finale Pfal supérieure à la pression atmosphérique Patm, le réservoir 102 est placé au dessus de la chambre 103 par rapport à l'axe longitudinale de la chambre 103, ici une portion de tube cylindrique : grâce à la pression de la matière du réservoir 102 du à la position du réservoir 102, la pression dans la chambre 103 passe de la pression initiale Pi, inférieure à la pression atmosphérique Patm, à la pression finale Pfalc supérieure à la pression atmosphérique Patm. Alternativement, on peut aussi utiliser une pompe péristaltique branchée sur le réservoir.

La dose est prélevée du réservoir 103 de façon similaire au dispositif 1 selon le premier mode de réalisation.

Une fois que la dose est dans la chambre 103, le module de commande ferme la vanne d'entrée 107, ouvre la vanne de pression 120 et la vanne de sortie 108 : la surpression à Pf dans la chambre accélère la sortie de la dose vers le récipient 103. Une fois que la chambre 103 est revenue à pression atmosphérique, la dose sort de la chambre grâce à la force de gravité.

Ainsi, il est possible d'utiliser un dispositif simplifié, qui comprenant un nombre minimal d'élément, notamment en s'affranchissant de la source de pression, pour préparer une dose de matière. Ce dispositif permet aussi de travailler à des pressions finales Pf supérieure à la pression atmosphérique Patm. Il est aussi possible de s'affranchir d'une source de vide si la pression initiale est égale à la pression atmosphérique.

Dans un mode de réalisation non représenté du dispositif selon le premier ou le deuxième mode de réalisation, le conduit de vide 13, 113 peut comprendre une chambre de vide, la dite chambre de vide étant connecté de manière réversible à une source de vide. La chambre de vide est une enceinte mise à une pression déterminé par la source ce vide. Puis, la chambre de vide est isolée de la source de vide.

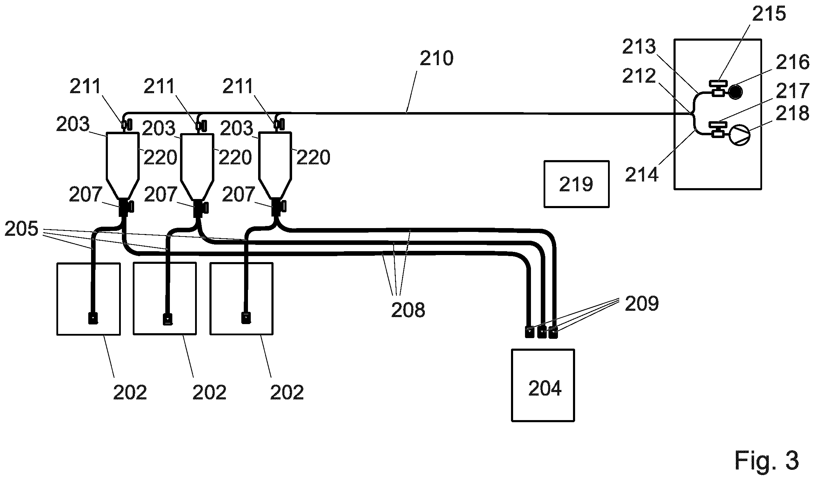

La figure 3 représente un système 200 selon l'invention, pour préparer une composition. Le système 200 est basé sur un dispositif 1 tel que représenté sur la figure 1. Le système 200 comprend trois réservoirs 211, chaque réservoir 202 comprend une vanne d'aspiration 206 est en liaison fluidique avec une chambre 203. Les trois chambres 203 sont en liaison fluidique avec un récipient 204.

La liaison fluidique entre chaque réservoir 203 et sa chambre 204 comprend un conduit d'entrée 205 avec une vanne d'aspiration 206 à l'extrémité dans le réservoir 203, et une vanne d'entrée 207 triple voie comme celle décrite au premier mode de réalisation.

La liaison fluidique entre chaque chambre 203 et le récipient comprend un conduit de sortie 208 avec la vanne d'entrée 207 à proximité de la chambre 203 et une vanne de sortie 209 par conduit de sortie 208.

Chaque chambre 3 est connectée à un conduit d'air 210, le conduit d'air 210 comprenant une vanne d'air 211 par chambre 203 dont l'ouverture ou la fermeture régule le flux d'entrée d'air de chaque chambre 203. Le système comprend un raccord 212, un conduit de vide 213, un conduit de pression 214, une vanne de vide 215, une source de vide 216 pour diminuer la pression dans chaque chambre 203 comme décrit dans le premier mode de réalisation. Grace à la présence d'une vanne d'air 211 par chambre 203, chaque chambre 203peut être mise à la pression désirée par la source de vide 216.

Le conduit de pression 214 comprend une vanne de pression 217, une source de pression 218 pour augmenter la pression dans chaque chambre 203 comme décrit dans le premier mode de réalisation. Grace à la présence d'une vanne d'air 211 par chambre 203, chaque chambre 203 peut être mise à la pression désirée par la source de pression 218.

Le système 200 comprend un module de commande 219. Le module de commande 219 est connecté à chaque capteur de pression 220 de chaque chambre 203 qui mesure la pression dans chaque chambre 203. Dans ce mode de réalisation, le module de commande 219 contrôle également les vannes d'entrée 207, les vannes d'air 211, la source de vide 226 et la vanne de vide 217, la source de pression 218 et la vanne de pression 217 (les connections n'ont pas été représentées sur la figure 3 pour faciliter la lecture de la figure).

Chaque réservoir 203 comprend une matière différente. L'utilisateur entre dans le module de commande 219 la formule de la composition, notamment la proportion de chaque matière, la masse de chaque matière dans la composition, la densité des matières, le volume de chaque chambre 203. Ensuite, le module de commande 219 calcule la pression finale Pfal pour préparer la dose de chaque matière dans chaque réservoir 202. Puis le module de commande exécute les étapes nécessaire pour prélever les doses, comme décrit dans le premier mode de réalisation, transféré lesdites doses dans le récipient 204. Ainsi, le système 200 selon l'invention facilite la préparation de composition comprenant plusieurs doses de différentes matières, dans un procédé qui implique un minimum de composants, et manière automatique, ce qui est économiquement intéressant.

### Numéros de référence employés sur les figures

- 1: Dispositif selon un premier mode de réalisation
- 2: Réservoir
- 3: Chambre
- 4: Récipient
- 5: Conduit d'entrée
- 6: Vanne d'aspiration
- 7: Vanne d'entrée
- 8: Conduit de sortie
- 9: Vanne de sortie
- 10: Conduit d'air
- 11: Vanne d'air
- 12: raccord
- 13: Conduit de vide
- 14: Conduit de pression
- 15: Vanne de vide
- 16: Source de vide
- 17: Vanne de pression
- 18: Source de pression
- 19: Module de commande
- 20: Capteur de pression
- 21: Limiteur de débit
- 22: Bille flottante
- 23: Clapet anti retour

- 100: Dispositif selon un deuxième mode de réalisation
- 102: réservoir
- 103: chambre
- 104: récipient
- 105: Conduit d'entrée
- 107: Vanne d'entrée
- 106: Vanne du récipient
- 110: Conduit d'air
- 111: Vanne d'air

- 112: raccord
- 113: Conduit de vide
- 114: Conduit de pression
- 115: Vanne de vide
- 117: Vanne de pression
- 119: Module de commande
- 120: Capteur de pression

- 200: Système selon l'invention
- 202: Réservoir
- 203: Chambre
- 204: Récipient
- 205: Conduit d'entrée
- 206: Vanne d'aspiration
- 207: Vanne d'entrée
- 208: Conduit de sortie
- 209: Vanne de sortie
- 210: Conduit d'air
- 211: Vanne d'air
- 212: raccord
- 213: Conduit de vide
- 214: Conduit de pression
- 215: Vanne de vide
- 216: Source de vide
- 217: Vanne de pression
- 218: Source de pression
- 219: Module de commande
- 220: Capteur de pression

## Revendications

1. Dispositif (1, 100) pour préparer une dose de matière, le dispositif (1, 100) comprenant:
- un réservoir (2, 102, 202) contenant la matière, le réservoir (2, 102, 202) étant en liaison fluidique avec une chambre (3, 103, 203) pour doser la matière, la chambre (3, 103, 203) étant en liaison fluidique avec un récipient (4, 104, 204) destiné à recevoir la dose de matière, de sorte que la matière à doser transite par la chambre (3, 103, 203) entre le réservoir (2, 102, 202) et le récipient (4, 104, 204),
- le dispositif (1, 100) comprenant une source de vide (16, 116, 216) pour établir une pression initiale Pi dans la chambre (3, 103, 203), et un capteur de pression (20, 120, 220) pour mesurer la pression dans la chambre (3, 103, 203), l'entrée de matière dans la chambre (3, 103, 203) induisant une variation de la pression dans la chambre (3, 103, 203) de la pression initiale Pi à une pression finale Pf, la différence entre la pression initiale Pi et la pression finale Pf correspondant, à température constante, à la dose de matière,
- le dispositif (1, 100) comprenant un module de commande (19, 119, 219) connecté au capteur de pression (20, 120, 220),
**caractérisé en ce que** ledit module (19, 119, 219) contrôle la liaison fluidique entre la chambre (3, 103, 203) et le réservoir (2, 102, 202), de façon à réguler l'introduction de matière dans la chambre (3, 103, 203), en fonction de la pression dans la chambre (3, 103, 203) mesurée par le capteur de pression (20, 120, 220), où le module de commande limite l'introduction de matière dans la chambre à la quantité nécessaire pour faire passer la pression de la chambre (3, 103, 203) de la pression initiale Pi à la pression finale Pf, et où le module de commande contrôle l'entrée de matière pour ajuster la pression de la chambre à la pression finale Pf.

2. Dispositif (1, 100) selon la revendication 1, dans lequel le module de commande (19, 119, 219) calcule soit la pression finale Pf à atteindre dans la chambre (3, 103, 203), soit la pression initiale Pi à établir.

3. Dispositif (1, 100) selon l'une des revendications 1 ou 2, dans lequel la liaison fluidique entre le réservoir (2, 102, 202) et la chambre (3, 103, 203) comprend une vanne d'entrée (7, 107, 207) qui présente un temps de commutation t1 entre une position ouverte et une position fermée, le module de commande (19, 119, 219) contrôle l'ouverture et la fermeture de ladite vanne (7, 107, 207), ledit module (19, 119, 219) tenant compte dudit temps de commutation t1 pour que la chambre (3, 103, 203) atteigne la pression finale Pf.

4. Dispositif (1, 100) selon l'une des revendications 1 à 3, dans lequel la liaison fluidique entre la chambre (3, 103, 203) et le réservoir (2, 102, 202) comprend un limiteur de débit (21), ledit limiteur (21) étant soit manuel soit contrôlé par le module de commande (19, 119, 219).

5. Dispositif (1, 100) selon l'une des revendications 1 à 4, dans lequel le dispositif (1, 100) comprend une source de pression (18, 218) reliée à la chambre (3, 103, 203), la source de pression (18, 218) étant soit manuelle soit contrôlée par le module de commande (19, 119, 219).

6. Dispositif (1, 100) selon l'une des revendications 1 à 5, dans lequel la liaison fluidique entre le réservoir (2, 102, 202) et la chambre (3, 103, 203) et/ou la liaison fluidique entre la chambre (3, 103, 203) et le récipient (4, 104, 204) comprend un module anti retour (23), ledit module anti retour (23) étant soit manuel soit contrôlé par le module de commande (19, 119, 219), par exemple une bille flottante, un clapet ou une vanne.

7. Dispositif (1, 100) selon l'une des revendications 1 à 6, le dispositif (1, 100) étant adapté à la préparation de dose de matière corrosive.

8. Procédé pour préparer une dose de matière, le procédé comprenant les étapes suivantes :
- i) Fournir un dispositif (1, 100) de dosage tel que défini dans les revendications 1 à 7,
- si la pression initiale Pi est prédéterminée,
o ii)a) Etablir la pression initiale Pi dans la chambre (3, 103, 203) et mesurer ladite pression initiale Pi de la chambre (3, 103, 203) avec le capteur de pression (20, 120, 220), la pression initiale Pi correspondant à la pression dans la chambre (3, 103, 203) avant l'introduction de la matière ;
o ii) b) Déterminer la dose de matière à préparer;
o ii) c) Calculer la pression finale Pf à atteindre dans la chambre (3, 103, 203) pour préparer ladite dose ;
- ou, si la pression finale Pf est prédéterminée,
o ii)d) Déterminer la dose de matière à préparer;
o ii)e) Calculer la pression initiale Pi à établir dans la chambre (3, 103, 203) pour préparer la dose en fonction de la pression finale Pf à atteindre dans la chambre (3, 103, 203), la pression finale Pf correspondant à la pression dans la chambre après l'introduction de la dose;
o ii)f) Etablir la pression initiale Pi dans la chambre (3, 103, 203) et mesurer ladite pression initiale Pi de la chambre (3, 103, 203) ;
- iii) Transférer de la matière à doser du réservoir (2, 102, 202) vers la chambre (3, 103, 203) via la liaison fluidique jusqu'à ce que la pression de la chambre (3, 103, 203) atteigne la pression finale Pf de façon à introduire dans la chambre (3, 103, 203) la dose de matière.

9. Procédé selon la revendication 8 comprenant l'étape suivante :
- Calibrer la chambre (3, 103, 203) pour déterminer la variation du volume de la chambre (3, 103, 203) en fonction de la variation de la pression dans chambre (3, 103, 203).

10. Procédé selon l'une des revendications 8 ou 9 comprenant l'étape suivante :
- Calculer la pression finale Pf à atteindre dans la chambre (3, 103, 203) pour préparer la dose, en fonction de la densité de la matière à doser, de la pression initiale Pi, du volume Vi de la chambre (3, 103, 203) ; ou
- calculer la pression initiale Pi à établir dans la chambre (3, 103, 203) pour préparer la dose, en fonction de la densité de la matière à doser, de la pression finale Pf, du volume Vi de la chambre (3, 103, 203).

11. Procédé selon l'une des revendications 8 à 10 dans lequel la chambre (3, 103, 203) est en liaison fluidique avec un récipient (4, 104, 204) destiné à recevoir la dose de matière, de sorte que la matière à doser transite par la chambre (3, 103, 203) entre le réservoir (2, 102, 202) et le récipient (4, 104, 204), le procédé comprenant l'étape suivante :
- Transférer la dose de matière depuis la chambre (3, 103, 203) vers le récipient (4, 104, 204).

12. Procédé selon l'une des revendications 8 à 11, dans lequel la liaison fluidique entre le réservoir (2, 102, 202) et la chambre (3, 103, 203) comprend une vanne d'entrée (7, 107, 207) qui présente un temps de commutation t1 entre une position ouverte et une position fermée, l'étape v) comprenant :
- adapter l'actionnement de la vanne d'entrée (7, 107, 207) en fonction du temps de commutation t1 pour introduire de la matière à doser dans la chambre (3, 103, 203) jusqu'à ce que la pression de la chambre (3, 103, 203) atteigne la pression finale Pf.

13. Procédé selon l'une des revendications 8 à 12, le dispositif (1, 100) comprenant une source de pression (18, 218) reliée à la chambre (3, 103, 203), par exemple un compresseur, le procédé comprenant l'étape suivante :
- Actionner la source de pression (18, 218) pour fournir un flux de gaz, par exemple de l'air, dans la chambre (3, 103, 203) et transférer la dose depuis la chambre (3, 103, 203) vers le récipient (4, 104, 204).

14. Procédé selon l'une des revendications 8 à 13, le procédé comprenant:
- Etablir une pression initiale Pi dans la chambre (3, 103, 203) en ajustant la pression dans la chambre (3, 103, 203) en plusieurs étapes pour atteindre la pression initiale, chaque étape étant espacé d'au moins 1 seconde; et/ou
- Transférer de la matière à doser du réservoir (2, 102, 202) vers dans la chambre (3, 103, 203), le transfert étant réalisé par palier, chaque palier étant séparé d'au moins 1 seconde.

15. Procédé selon l'une des revendications 8 à 14, comprenant une étape de programmation d'un module de commande (19, 119, 219) pour exécuter de façon automatique au moins l'une des étapes suivantes :
- si la pression initiale Pi est prédéterminée,
o Etablir la pression initiale Pi dans la chambre (3, 103, 203) et mesurer ladite pression initiale Pi de la chambre (3, 103, 203) avec le capteur de pression (20, 120, 220), la pression initiale Pi correspondant à la pression dans la chambre (3, 103, 203) avant l'introduction de la matière ;
∘ Déterminer la dose de matière à préparer;
∘ Calculer la pression finale Pf à atteindre dans la chambre (3, 103, 203) pour préparer ladite dose ;
- ou, si la pression finale Pf est prédéterminée,
∘ Déterminer la dose de matière à préparer;
∘ Calculer la pression initiale Pi à établir dans la chambre (3, 103, 203) pour préparer la dose en fonction de la pression finale Pf à atteindre dans la chambre (3, 103, 203), la pression finale Pf correspondant à la pression dans la chambre après l'introduction de la dose;
∘ Etablir la pression initiale Pi dans la chambre (3, 103, 203) et mesurer ladite pression initiale Pi de la chambre (3, 103, 203) ;
- Transférer de la matière à doser du réservoir (2, 102, 202) vers dans la chambre (3, 103, 203) via la liaison fluidique jusqu'à ce que la pression de la chambre (3, 103, 203) atteigne la pression finale Pf de façon à introduire dans la chambre (3, 103, 203) la dose de matière ;
- Calibrer la chambre (3, 103, 203) pour déterminer la variation du volume de la chambre (3, 103, 203) en fonction de la variation de la pression dans chambre (3, 103, 203);
- Déterminer une pression finale Pf à atteindre dans la chambre (3, 103, 203) pour préparer la dose en fonction de la densité de la matière à doser, de la pression initiale Pi et du volume Vi de la chambre (3, 103, 203) ;
- Transférer la dose de matière depuis la chambre (3, 103, 203) vers le récipient (4, 104, 204) ;
- Adapter l'actionnement de la vanne d'entrée (7, 107, 207) en fonction du temps de commutation t1 pour introduire de la matière à doser dans la chambre (3, 103, 203) jusqu'à ce que la pression de la chambre (3, 103, 203) atteigne la pression finale Pf;
- Actionner la source de pression (18, 218) pour fournir un flux de gaz, par exemple de l'air, dans la chambre (3, 103, 203) et transférer la dose depuis la chambre (3, 103, 203) vers le récipient (4, 104, 204) ;
- Etablir une pression initiale Pi dans la chambre (3, 103, 203) en ajustant la pression dans la chambre (3, 103, 203) en plusieurs étapes pour atteindre la pression initiale, chaque étape étant espacé d'au moins 1 seconde; et/ou
- Transférer de la matière à doser du réservoir (4, 104, 204) vers dans la chambre (3, 103, 203), le transfert étant réalisé par palier, chaque palier étant séparé d'au moins 1 seconde.

## Patentansprüche

1. Vorrichtung (1, 100) zur Vorbereitung einer Stoffdosis, die Vorrichtung (1, 100) umfassend:
- einen Vorratsbehälter (2, 102, 202), der den Stoff enthält, wobei der Vorratsbehälter (2, 102, 202) mit einer Kammer (3, 103, 203) in Fluidverbindung steht, um den Stoff zu dosieren, wobei die Kammer (3, 103, 203) mit einem Behältnis (4, 104, 204) in Fluidverbindung steht, das dazu bestimmt ist, die Stoffdosis aufzunehmen, so dass der zu dosierende Stoff durch die Kammer (3, 103, 203) zwischen dem Vorratsbehälter (2, 102, 202) und dem Behältnis (4, 104, 204) hindurchtritt,
- wobei die Vorrichtung (1, 100) eine Vakuumquelle (16, 116, 216) zum Aufbau eines Anfangsdrucks Pi in der Kammer (3, 103, 203) und einen Drucksensor (20, 120, 220) zur Messung des Drucks in der Kammer (3, 103, 203) umfasst, wobei der Stoffeintritt in die Kammer (3, 103, 203) eine Änderung des Drucks in der Kammer (3, 103, 203) vom Anfangsdruck Pi zu einem Enddruck Pf verursacht, wobei die Differenz zwischen dem Anfangsdruck Pi und dem Enddruck Pf bei konstanter Temperatur der Stoffdosis entspricht,
- wobei die Vorrichtung (1, 100) ein Steuerungsmodul (19, 119, 219) umfasst, das an den Drucksensor (20, 120, 220) angeschlossen ist,
**dadurch gekennzeichnet, dass** das Modul (19, 119, 219) die Fluidverbindung zwischen der Kammer (3, 103, 203) und dem Vorratsbehälter (2, 102, 202) so steuert, dass die Einleitung von Stoff in die Kammer (3, 103, 203) in Abhängigkeit vom Druck in der Kammer (3, 103, 203) reguliert wird, der vom Drucksensor (20, 120, 220) gemessen wird, wobei das Steuerungsmodul die Einleitung von Stoff in die Kammer auf die Menge begrenzt, die notwendig ist, um den Druck der Kammer (3, 103, 203) vom Anfangsdruck Pi auf den Enddruck Pf zu bringen, und wobei das Steuerungsmodul den Stoffeintritt steuert, um den Druck der Kammer auf den Enddruck Pf einzustellen.

2. Vorrichtung (1, 100) nach Anspruch 1, wobei das Steuerungsmodul (19, 119, 219) entweder den Enddruck Pf, der in der Kammer (3, 103, 203) zu erreichen ist, oder den aufzubauenden Anfangsdruck Pi berechnet.

3. Vorrichtung (1, 100) nach einem der Ansprüche 1 oder 2, wobei die Fluidverbindung zwischen dem Vorratsbehälter (2, 102, 202) und der Kammer (3, 103, 203) ein Einlassventil (7, 107, 207) umfasst, das eine Umschaltzeit t1 zwischen einer offenen Position und einer geschlossenen Position aufweist, das Steuerungsmodul (19, 119, 219) das Öffnen und Schließen des Ventils (7, 107, 207) steuert, wobei das Modul (19, 119, 219) die Umschaltzeit t1 berücksichtigt, damit die Kammer (3, 103, 203) den Enddruck Pf erreicht.

4. Vorrichtung (1, 100) nach einem der Ansprüche 1 bis 3, wobei die Fluidverbindung zwischen der Kammer (3, 103, 203) und dem Vorratsbehälter (2, 102, 202) einen Durchflussbegrenzer (21) umfasst, wobei der Begrenzer (21) entweder manuell ist oder vom Steuerungsmodul (19, 119, 219) gesteuert wird.

5. Vorrichtung (1, 100) nach einem der Ansprüche 1 bis 4, wobei die Vorrichtung (1, 100) eine Druckquelle (18, 218) umfasst, die mit der Kammer (3, 103, 203) verbunden ist, wobei die Druckquelle (18, 218) entweder manuell ist oder vom Steuerungsmodul (19, 119, 219) gesteuert wird.

6. Vorrichtung (1, 100) nach einem der Ansprüche 1 bis 5, wobei die Fluidverbindung zwischen dem Vorratsbehälter (2, 102, 202) und der Kammer (3, 103, 203) und/oder die Fluidverbindung zwischen der Kammer (3, 103, 203) und dem Behältnis (4, 104, 204) ein Rücklaufschutzmodul (23) umfasst, wobei das Rücklaufschutzmodul (23) entweder manuell ist oder vom Steuerungsmodul (19, 119, 219) gesteuert wird, zum Beispiel eine schwimmende Kugel, eine Klappe oder ein Ventil.

7. Vorrichtung (1, 100) nach einem der Ansprüche 1 bis 6, wobei die Vorrichtung (1, 100) zur Vorbereitung einer Dosis korrosiven Stoffs geeignet ist.

8. Verfahren zur Vorbereitung einer Stoffdosis, wobei das Verfahren die folgenden Schritte umfasst:
- i) Bereitstellen einer Dosierungsvorichtung (1, 100) nach den Ansprüchen 1 bis 7,
- wenn der Anfangsdruck Pi vorbestimmt ist,
o ii)a) Aufbauen des Anfangsdrucks Pi in der Kammer (3, 103, 203) und Messen des Anfangsdrucks Pi der Kammer (3, 103, 203) mit dem Drucksensor (20, 120, 220), wobei der Anfangsdruck Pi dem Druck in der Kammer (3, 103, 203) vor der Einleitung des Stoffs entspricht;
∘ ii) b) Bestimmen der vorzubereitenden Stoffdosis;
∘ ii) c) Berechnen des Enddrucks Pf, der in der Kammer (3, 103, 203) zu erreichen ist, um die Dosis vorzubereiten;
- oder, wenn der Enddruck Pf vorbestimmt ist,
∘ ii)d) Bestimmen der vorzubereitenden Stoffdosis;
∘ ii)e) Berechnen des Anfangsdrucks Pi, der in der Kammer (3, 103, 203) aufzubauen ist, um die Dosis vorzubereiten, in Abhängigkeit vom Enddruck Pf, der in der Kammer (3, 103, 203) zu erreichen ist, wobei der Enddruck Pf dem Druck in der Kammer nach der Einleitung der Dosis entspricht;
∘ ii)f) Aufbauen des Anfangsdrucks Pi in der Kammer (3, 103, 203) und Messen des Anfangsdrucks Pi der Kammer (3, 103, 203);
- iii) Übertragen von zu dosierendem Stoff vom Vorratsbehälter (2, 102, 202) zur Kammer (3, 103, 203) über die Fluidverbindung, bis der Druck der Kammer (3, 103, 203) den Enddruck Pf erreicht, um die Stoffdosis in die Kammer (3, 103, 203) einzuleiten.

9. Verfahren nach Anspruch 8, umfassend den folgenden Schritt:
- Kalibrieren der Kammer (3, 103, 203), um die Änderung des Volumens der Kammer (3, 103, 203) in Abhängigkeit von der Änderung des Drucks in der Kammer (3, 103, 203) zu bestimmen.

10. Verfahren nach einem der Ansprüche 8 oder 9, umfassend den folgenden Schritt:
- Berechnen des Enddrucks Pf, der in der Kammer (3, 103, 203) zu erreichen ist, um die Dosis vorzubereiten, in Abhängigkeit von der Dichte des zu dosierenden Stoffs, dem Anfangsdruck Pi, dem Volumen Vi der Kammer (3, 103, 203); oder
- Berechnen des Anfangsdrucks Pi, der in der Kammer (3, 103, 203) aufzubauen ist, um die Dosis vorzubereiten, in Abhängigkeit von der Dichte des zu dosierenden Stoffs, dem Enddruck Pf, dem Volumen Vi der Kammer (3, 103, 203).

11. Verfahren nach einem der Ansprüche 8 bis 10, wobei die Kammer (3, 103, 203) mit einem Behältnis (4, 104, 204) in Fluidverbindung steht, das dazu bestimmt ist, die Stoffdosis aufzunehmen, so dass der zu dosierende Stoff durch die Kammer (3, 103, 203) zwischen dem Vorratsbehälter (2, 102, 202) und dem Behältnis (4, 104, 204) hindurchtritt, wobei das Verfahren den folgenden Schritt umfasst:
- Übertragen der Stoffdosis von der Kammer (3, 103, 203) zum Behältnis (4, 104, 204).

12. Verfahren nach einem der Ansprüche 8 bis 11, wobei die Fluidverbindung zwischen dem Vorratsbehälter (2, 102, 202) und der Kammer (3, 103, 203) ein Einlassventil (7, 107, 207) umfasst, das eine Umschaltzeit t1 zwischen einer offenen Position und einer geschlossenen Position aufweist, wobei der Schritt v) Folgendes umfasst:
- Anpassen der Betätigung des Einlassventils (7, 107, 207) in Abhängigkeit von der Umschaltzeit t1 zur Einleitung des zu dosierenden Stoffs in die Kammer (3, 103, 203), bis der Druck der Kammer (3, 103, 203) den Enddruck Pf erreicht.

13. Verfahren nach einem der Ansprüche 8 bis 12, wobei die Vorrichtung (1, 100) eine Druckquelle (18, 218) umfasst, die mit der Kammer (3, 103, 203) verbunden ist, zum Beispiel einen Kompressor, wobei das Verfahren den folgenden Schritt umfasst:
- Betätigen der Druckquelle (18, 218), um einen Gasstrom, zum Beispiel Luft, in der Kammer (3, 103, 203) bereitzustellen und die Dosis von der Kammer (3, 103, 203) zum Behältnis (4, 104, 204) zu übertragen.

14. Verfahren nach einem der Ansprüche 8 bis 13, das Verfahren umfassend:
- Aufbauen eines Anfangsdrucks Pi in der Kammer (3, 103, 203) durch Einstellung des Drucks in der Kammer (3, 103, 203) in mehreren Schritten, um den Anfangsdruck zu erreichen, wobei jeder Schritt um mindestens 1 Sekunde beabstandet ist; und/oder
- Übertragen des zu dosierenden Stoffs vom Vorratsbehälter (2, 102, 202) zur Kammer (3, 103, 203), wobei die Übertragung stufenweise durchgeführt wird, wobei jede Stufe um mindestens 1 Sekunde getrennt ist.

15. Verfahren nach einem der Ansprüche 8 bis 14, umfassend einen Schritt des Programmierens eines Steuerungsmoduls (19, 119, 219) zur automatischen Ausführung mindestens eines der folgenden Schritte:
- wenn der Anfangsdruck Pi vorbestimmt ist,
∘ Aufbauen des Anfangsdrucks Pi in der Kammer (3, 103, 203) und Messen des Anfangsdrucks Pi der Kammer (3, 103, 203) mit dem Drucksensor (20, 120, 220), wobei der Anfangsdruck Pi dem Druck in der Kammer (3, 103, 203) vor der Einleitung des Stoffs entspricht;
∘ Bestimmen der vorzubereitenden Stoffdosis;
o Berechnen des Enddrucks Pf, der in der Kammer (3, 103, 203) zu erreichen ist, um die Dosis vorzubereiten;
- oder, wenn der Enddruck Pf vorbestimmt ist,
∘ Bestimmen der vorzubereitenden Stoffdosis;
∘ Berechnen des Anfangsdrucks Pi, der in der Kammer (3, 103, 203) aufzubauen ist, um die Dosis vorzubereiten, in Abhängigkeit vom Enddruck Pf, der in der Kammer (3, 103, 203) zu erreichen ist, wobei der Enddruck Pf dem Druck in der Kammer nach der Einleitung der Dosis entspricht;
∘ Aufbauen des Anfangsdrucks Pi in der Kammer (3, 103, 203) und Messen des Anfangsdrucks Pi der Kammer (3, 103, 203) ;
- Übertragen von zu dosierendem Stoff vom Vorratsbehälter (2, 102, 202) zur Kammer (3, 103, 203) über die Fluidverbindung, bis der Druck der Kammer (3, 103, 203) den Enddruck Pf erreicht, um die Stoffdosis in die Kammer (3, 103, 203) einzuleiten;
- Kalibrieren der Kammer (3, 103, 203), um die Änderung des Volumens der Kammer (3, 103, 203) in Abhängigkeit von der Änderung des Drucks in der Kammer (3, 103, 203) zu bestimmen;
- Bestimmen eines Enddrucks Pf, der in der Kammer (3, 103, 203) zu erreichen ist, um die Dosis vorzubereiten, in Abhängigkeit von der Dichte des zu dosierenden Stoffs, dem Anfangsdruck Pi und dem Volumen Vi der Kammer (3, 103, 203);
- Übertragen der Stoffdosis von der Kammer (3, 103, 203) zum Behältnis (4, 104, 204);
- Anpassen der Betätigung des Einlassventils (7, 107, 207) in Abhängigkeit von der Umschaltzeit t1 zur Einleitung des zu dosierenden Stoffs in die Kammer (3, 103, 203), bis der Druck der Kammer (3, 103, 203) den Enddruck Pf erreicht;
- Betätigen der Druckquelle (18, 218), um einen Gasstrom, zum Beispiel Luft, in der Kammer (3, 103, 203) bereitzustellen und die Dosis von der Kammer (3, 103, 203) zum Behältnis (4, 104, 204) zu übertragen;
- Aufbauen eines Anfangsdrucks Pi in der Kammer (3, 103, 203) durch Einstellung des Drucks in der Kammer (3, 103, 203) in mehreren Schritten, um den Anfangsdruck zu erreichen, wobei jeder Schritt um mindestens 1 Sekunde beabstandet ist; und/oder
- Übertragen des zu dosierenden Stoffs vom Vorratsbehälter (4, 104, 204) zur Kammer (3, 103, 203), wobei die Übertragung stufenweise durchgeführt wird, wobei jede Stufe um mindestens 1 Sekunde getrennt ist.

## Claims

1. Device (1, 100) for preparing a dose of material, the device (1, 100) comprising:
- a reservoir (2, 102, 202) containing the material, the reservoir (2, 102, 202) being in fluidic communication with a chamber (3, 103, 203) for dosing the material, the chamber (3, 103, 203) being in fluidic communication with a container (4, 104, 204) adapted for receiving the dose of material, so that the material to be dosed passes through the chamber (3, 103, 203) between the reservoir (2, 102, 202) and the container (4, 104, 204),
- the device (1, 100) comprising a vacuum source (16, 116, 216) for establishing an initial pressure Pi in the chamber (3, 103, 203), and a pressure sensor (20, 120, 220) for measuring the pressure in the chamber (3, 103, 203), the material entry in the chamber (3, 103, 203) inducing a pressure variation in the chamber (3, 103, 203) from the initial pressure Pi to a finale pressure Pf, corresponding to, at constant temperature, the dose of material,
- the device (1, 100) comprising a command module (19, 119, 219) connected to the pressure sensor (20, 120, 220).
**characterized in that** said module (19, 119, 219) controls the fluidic communication between the chamber (3, 103, 203) and the reservoir (2, 102, 202), so as to regulate the entry of material in the chamber (3, 103, 203), according to the pressure in the chamber (3, 103, 203) measured by means of the pressure sensor (20, 120, 220), wherein the command module limits the introduction of material in the chamber to the amount necessary for varying the pressure in the chamber (3, 103, 203) from the initial pressure Pi to the final pressure Pf, and wherein the command module controls the material entry to adjust the pressure in the chamber at the final pressure Pf.

2. Device (1, 100) according to claim 1, wherein the command module (19, 119, 219) calculates either the final pressure Pf to be reached in the chamber (3, 103, 203) or the initial pressure Pi to be established.

3. Device (1, 100) according to one of claims 1 or 2, wherein the fluidic communication between the reservoir (2, 102, 202) and the chamber (3, 103, 203) comprises an input valve (7, 107, 207) which presents a switching time t1 between an open position and a closed position, the command module (19, 119, 219) controls the opening and the closing of said valve (7, 107, 207), said module (19, 119, 219) considering said switching time t1 so that the chamber (3, 103, 203) reaches the final pressure Pf.

4. Device (1, 100) according to one of claims 1 to 3, wherein the fluidic communication between the chamber (3, 103, 203) and the reservoir (2, 102, 202) comprises a rate of flow limiting means (21), said rate of flow limiting means (21) being either manual or controlled by the command module (19, 119, 219).

5. Device (1, 100) according to one of claims 1 to 4, wherein the device (1, 100) comprises a pressure source (18, 218) connected to the chamber (3, 103, 203), the pressure source (18, 218) being either manual or controlled by the command module (19, 119, 219).

6. Device (1, 100) according to one of claims 1 to 5, wherein the fluidic communication between the reservoir (2, 102, 202) and the chamber (3, 103, 203) and/or the fluidic communication between the chamber (3, 103, 203) and the container (4, 104, 204) comprises a non-return module (23), said non-return module (23) being either manual or controlled by the command module (19, 119, 219), for example a floating ball, a gate or a valve.

7. Device (1, 100) according to one of claims 1 to 6, the device (1, 100) being adapted for the preparation of a dose of a corrosive material.

8. Process for preparing a dose of material, the process comprising the following steps:
- i) providing a device (1, 100) for dosing, as defined in claims 1 to 7,
- if the initial pressure Pi is determined,
o ii) a) establishing the initial pressure Pi in the chamber (3, 103, 203) and measuring said initial pressure Pi of the chamber (3, 103, 203) with the pressure sensor (20, 120, 220), the initial pressure Pi corresponding to the pressure in the chamber (3, 103, 203) before introducing the material;
o ii) b) determining the dose of material to be prepared;
o ii) c) calculating the final pressure Pf to be reached in the chamber (3, 103, 203) to prepare the dose;
- or, if the final pressure Pf is determined,
o ii) d) determining the dose of material to be prepared;
o ii) e) calculating the initial pressure to be established in the chamber (3, 103, 203) for preparing the dose according to the final pressure Pf to be reached in the chamber (3, 103, 203), the final pressure Pf corresponding to the pressure in the chamber after introducing the dose;
o ii) f) establishing the initial pressure Pi in the chamber (3, 103, 203) and measuring said initial pressure Pi of the chamber (3, 103, 203);
- iii) transferring the material to be dosed from the reservoir (2, 102, 202) to the chamber (3, 103, 203) via the fluidic communication until the pressure of the chamber (3, 103, 203) reaches the final pressure Pf so as to introduce in the chamber (3, 103, 203) the dose of material.

9. Process according to claim 8 comprising the following step:
- calibrating the chamber (3, 103, 203) for determining the variation of volume of the chamber (3, 103, 203) according to the variation of the pressure in the chamber (3, 103, 203).

10. Process according to one of claims 8 or 9 comprising the following step:
- calculating the final pressure Pf to be reached in the chamber (3, 103, 203) for preparing the dose, according to the density of the material to be dosed, to the initial pressure Pi, to the volume Vi of the chamber (3, 103, 203); or
- calculating the initial pressure Pi to be established in the chamber (3, 103, 203) for preparing the dose, according to the density of the material to be dosed, to the final pressure Pf, to the volume Vi of the chamber (3, 103, 203).

11. Process according to one of claims 8 to 10, wherein the chamber (3, 103, 203) is in fluidic communication with a container (4, 104, 204) adapted to receive the dose of material, so that the material to be dosed passes through the chamber (3, 103, 203) between the reservoir (2, 102, 202) and the container (4, 104, 204), the process comprising the following step :
- transferring the dose of material from the chamber (3, 103, 203) to the container (4, 104, 204).

12. Process according to one of claims 8 to 11, wherein the fluidic communication between the reservoir (2, 102, 202) and the chamber (3, 103, 203) comprises an input valve (7, 107, 207) which presents a switching time t1 between an open position and a closed position, the step v) comprising:
- adapting the actuation of the input valve (7, 107, 207) according to the switching time t1 for introducing material to be dosed in the chamber (3, 103, 203) until the pressure of the chamber (3, 103, 203) reaches the final pressure Pf.

13. Process according to one of claims 8 to 12, the device (1, 100) comprising a pressure source (8, 218) connected to the chamber (3, 103, 203), for example a compressor, the process comprising the following step:
- actuating the pressure source (18, 218) to provide a gas flow, for example air, in the chamber (3, 103, 203) and transferring the dose from the chamber (3, 103, 203) to the container (4, 104, 204).

14. Process according to one of claims 8 to 13, the process comprising :
- establishing an initial pressure Pi in the chamber (3, 103, 203) by adjusting the pressure in the chamber (3, 103, 203) in several steps to reach the initial pressure, each step being spaced at least by 1 second; and/or
- transferring some material to be dosed from the reservoir (2, 102, 202) to the chamber (3, 103, 203), the transfer being realised by level, each level being separated by at least one second.

15. Process according to one of claims 1 to 14, comprising a step of programming the command module (19, 119, 219) for automatically executing at least one of the following steps:
- If the initial pressure Pi is determined,
o establishing the initial pressure Pi in the chamber (3, 103, 203) and measuring said initial pressure Pi of the chamber (3, 103, 203) with the pressure sensor (20, 120, 220), the initial pressure Pi corresponding to the pressure in the chamber (3, 103, 203) before introducing the material;
o determining the dose of material to be prepared;
∘ calculating the final pressure Pf to be reached in the chamber (3, 103, 203) for preparing said dose;
- or, if the final pressure Pf is determined,
∘ determining the dose of material to be prepared;
∘ calculating the initial pressure Pi to be established in the chamber (3, 103, 203) to prepare the dose according to the final pressure Pf to be reached in the chamber (3, 103, 203), the final pressure Pf corresponding to the pressure in the chamber after introducing the dose;
o establishing the initial pressure Pi in the chamber (3, 103, 203) and measuring said initial pressure Pi (3, 103, 203);
- transferring some material to be dosed from the reservoir (2, 102, 202) to the chamber (3, 103, 203) via the fluidic communication until the pressure of the chamber (3, 103, 203) reaches the final pressure Pf so as to introduce in the chamber (3, 103, 203) the dose of material;
- calibrating the chamber (3, 103, 203) for determining the variation of the volume of the chamber (3, 103, 203) according to the variation of the pressure in the chamber (3, 103, 203);
- determining a final pressure Pf to be reached in the chamber (3, 103, 203) for preparing the dose according to the density of the material to be dosed, to the initial pressure Pi and to the volume Vi of the chamber (3, 103, 203);
- transferring the dose of material from the chamber (3, 103, 203) to the container (4, 104, 204);
- adapting the actuation of the input valve (7, 107, 207) according to the switching time t1 for introducing some material to be dosed in the chamber (3, 103, 203) until the pressure of the chamber (3, 103, 203) reaches the final pressure Pf;
- actuating the pressure source (18, 218) to provide a gas flow, for example air, in the chamber (3, 103, 203) and transferring the dose from the chamber (3, 103, 203) to the container (4, 104, 204);
- establishing an initial pressure Pi in the chamber (3, 103, 203) by adjusting the pressure in the chamber (3, 103, 203) in several steps to reach the initial pressure, each step being spaced by at least one second, and/or
- transferring some material to be dosed from the reservoir (4, 104, 204) to the chamber (3, 103, 203), the transfer being realised by level, each level being spaced by at least one second.
